# EUROPÄISCHE PATENTSCHRIFT

(11) **EP 2 123 326 B1**
(45) Veröffentlichungstag und Bekanntmachung des Hinweises auf die Patenterteilung: **25.05.2011**
(21) Anmeldenummer: 09158448.2
(22) Anmeldetag: 22.04.2009
(51) Int. Cl.: A61N 1/39, A61M 5/142

(54) **Implantierbare Schockelektrodenleitung und implantierbare Defibrillationsanordnung**
Implantable shock electrode line and implantable defibrillation arrangement
Appareil de commande de direction

(30) Priorität: 20.05.2008 DE 102008024447
(43) Veröffentlichungstag der Anmeldung: 25.11.2009
(73) Patentinhaber: Biotronik CRM Patent AG, 6341 Baar (CH)
(72) Erfinder: Dörr, Thomas, 12437, Berlin (DE)
(74) Vertreter: Lindner-Vogt, Karin L.

(56) Entgegenhaltungen:
- US-A1- 2003 153 951
- US-A1- 2006 041 276
- US-A1- 2007 150 008

## Beschreibung

Die Erfindung betrifft eine implantierbare insbesondere endokardiale Schockelektrodenleitung sowie eine implantierbare. Sie betrifft des Weiteren eine implantierbare Kardioversions bzw. Defibrillationsanordnung.

Neben implantierbaren Schrittmacheranordnungen, die Stimulationsimpulse an das Herz eines Patienten über endokardiale Elektrodenleitungen mit distalen Elektroden abgeben, sind seit langem auch ähnlich aufgebaute implantierbare Kardioverter oder Defibrillatoren bekannt. Diese haben gegenüber externen Defibrillatoren, die bei Herzstillstand eines Patienten durch medizinisches Personal angewandt werden, den entscheidenden Vorteil der automatischen Inbetriebnahme aufgrund einer körperinternen Erfassung bedrohlicher Herzzustände des Patienten. Sie können damit vor allem praktisch verzögerungsfrei und unabhängig von der Anwesenheit von Helfern bei Kammerflimmern oder Herzstillstand des Patienten intervenieren.

Andererseits ist die Erfolgsrate implantierbarer Kardioverter bzw. Defibrillatoren signifikant geringer als diejenige extern angewandter Defibrillatoren. Dies ist zum einen auf die begrenzte Energiespeicherkapazität implantierbarer Geräte mit Batteriestromversorgung und zum anderen auf die durch eine endokardial oder epikardial verlegte Elektrodenleitung auferlegten Beschränkungen hinsichtlich der Beaufschlagung des Herzmuskelgewebes mit der elektrischen Energie der Schockimpulse bedingt.

Des Weiteren können die derzeitigen implantierbaren Kardioversions-(ICD)-Systeme nur dann einen Therapieerfolg erzielen, wenn die Ursache des Kammerflimmerns (VF) allein rhythmogen bedingt ist oder eine temporäre Störung der Myokardperfusion vorliegt. Die alleinige Defibrillation einer VF im Rahmen eines akuten Myokardinfarktes hingegen hat eher eine schlechte Prognose, da hier die Bedingungen für eine erneute VF nach Defibrillation unverändert gegeben sind.

Als wichtiges Anliegen zukünftiger ICD-Therapien wird daher in Expertengremien die Steigerung der Effizienz der Defibrillation formuliert. Die derzeitigen ICD-Systeme sind hinsichtlich der elektrischen Therapie weitgehend ausgereift und bieten nur geringfügiges Potential bei der gewünschten Steigerung der Effizienz der Defibrillation. Die zu enivartenden Verbesserungen werden aufgrund der benötigten Patientenzahlen nur schwer oder nicht für die klinischen Studien nachweisbar sein.

Beachtenswert ist in diesem Zusammenhang, dass bei der externen Defibrillation im Rahmen einer Reanimation immer auch medikamentöse Therapien ergänzend zur Defibrillation eingesetzt werden, um so die Effizienz der Defibrillation zu steigern (siehe z. B.: http/www.inmonline.de/pdf/Wissen/Reanimation/algorithmus 2005.pdf).

Implantierbare Anordnungen zur dosierten Medikamentenabgabe sind, etwa in Form von implantierbaren Insulinpumpen, seit langem bekannt und in gewissen Umfang auch im Einsatz. Derartige Anordnungen sind auch in den letzten Jahren Gegenstand intensiver Entwicklungsbemühungen gewesen.

So wird in der EP 1 210 064 B1 ein thermisch aktivierbarer Mikrochip als Abgabevorrichtung für Chemikalien, insbesondere Medikamente, vorgestellt und beansprucht, bei dem die Medikamentenfreisetzung aus einem Reservoir im Wesentlichen durch Widerstandserwärmung und darauf folgende Zerstörung einer Kappe des Reservoirs bewirkt wird. Die WO 2004/071487 A2 beschreibt ebenfalls eine Anordnung zur gesteuerten Medikamentenfreisetzung, bei der auf einem Substrat eine Vielzahl einzelner Reservoirs angeordnet ist und jedes davon durch elektrische Ansteuerung zur Freisetzung der darin enthaltenen Medikamentenmenge geöffnet werden kann. Hierin ist auch beschrieben, dass eine Anordnung solcher Medikamentenreservoirs am distalen Ende eines Katheters angeordnet und mit einer implantierbaren Ansteuereinheit verbunden ist.

Die WO 2004/033034 A1 beschreibt ein medizinisches Gerät zur Nervenstimulation und gesteuerten Medikamentenabgabe, welches neben einer implantierbaren Elektrodenleitung gleichfalls einen Mikrochip zur Medikamentenfreisetzung umfasst. Hierbei ist der Mikrochip mit einer Vielzahl einzeln ansteuerbarer Medikamentenreservoirs an einem Gerätegehäuse platziert, das eine Steuereinrichtung für die Medikamentenabgabe oder eine gemeinsame Steuereinrichtung für Medikamentenabgabe und elektrische NervenStimulation aufnimmt. Die WO 2004/033036 A2 beschreibt eine sehr ähnliche Anordnung, die jedoch zur koordinierten Medikamentenabgabe und Überwachung und/oder Stimulation des Herzens ausgebildet ist. In einer Ausführung weist die hierin beschriebene Anordnung auf entsprechenden Elektrodenleitungen auch Kardioversionselektroden auf, und es wird erwähnt, dass die Medikamentenfreisetzung aus den am Gerätegehäuse befindlichen Reservoirs mit gewissem zeitlichem Abstand vor der Ausgabe eines Kardioversions-Schockimpulses erfolgen kann.

Aus der US 2006/0041276 A1 sind ein Verfahren und eine Anordnung zur kombinierten Bereitstellung von Elektro- und Medikamententherapie bekannt, welche insbesondere zur Einstellung des Herzrhythmus vorgesehen sind. In verschiedenen Ausführungen umfasst die Anordnung ein miteinander gekoppeltes implantierbares Elektrostimulations- und implantierbares Medikamentenfreisetzungsgerät mit einem gemeinsamen Elektrodenkatheter oder zwei separaten Elektroden- bzw. Infusionsleitungen oder aber ein in einem einzelnen Gehäuse kombiniertes Elektrostimulations- und Medikamentenabgabegerät mit separaten Steuereinheiten oder einer gemeinsamen Steuereinheit und einem einzelnen Elektroden- und Medikamentenabgabekatheter. Es wird auch beschrieben, dass das implantierbare medizinische Gerät eine Kardioversions- bzw. Defibrillationseinheit und die Elektrodenleitung bzw. der Katheter Schockelektroden aufweisen kann.

Die insoweit bekannten Lösungen für eine koordinierte Elektrostimulation, insbesondere Kardioversion/Defibrillation, und Medikamentenabgabe sind in der technischen Ausführung relativ aufwändig und nicht ohne weiteres kompatibel mit etablierten ICD-Lösungen.

Es ist daher Aufgabe der Erfindung, eine verbesserte implantierbare Schockelektrodenanordnung und eine entsprechende Kardioversions-/Defibrillationsanordnung anzugeben, die vergleichsweise einfach und kostengünstig zu realisieren und zu etablierten ICD-Systemen kompatibel sind.

Diese Aufgabe wird durch eine implantierbare Schockelektrodenleitung mit den Merkmalen des Anspruchs 1 sowie durch eine implantierbare Defibrillationsanordnung mit den Merkmalen der Ansprüche gelöst Zweckmäßige Fortbildungen des Erfindungsgedankens sind Gegenstand der abhängigen Ansprüche.

Die Erfindung schließt den wesentlichen Gedanken ein, die zusätzlich zur Bereitstellung der Schockimpulse erforderlichen Mittel zur Medikamentenfreisetzung im Wesentlichen allein in der Elektrodenleitung anzuordnen, um mit einem an sich bekannten implantierbaren Kardioverter/Defibrillator eine neuartige Gesamtanordnung mit verbesserten Wirkungsmöglichkeiten bilden zu können. Sie schließt des Weiteren, mindestens in einer relativ selbstständigen Ausprägung des Erfindungsgedankens, die Überlegung ein, die Medikamentenfreisetzung im Wesentlichen direkt, ohne zusätzliche Ansteuermittel, durch die Ausgabe eines Kardioversions- bzw. Defibrillationsimpulses zu bewirken.

Die erfindungsgemäße Lösung erhöht durch die gleichzeitig zur Defibrillation abgegebenen Notfallmedikation des Patienten die Prognose für eine erfolgreiche Wiederbelebung des Patienten. Bei entsprechender Dosierung sind die Erfolgsraten ähnlich der bei externer Defibrillation und Medikation anzunehmen. Insbesondere bei ischämisch induziertem Kammerflimmern kann die zusätzliche Medikation einen Therapieerfolg erzielen. Die eingesetzte Elektrode bleibt dabei kompatibel zu existierenden ICDs.

Die erfindungsgemäße Schockelektrodenleitung zeichnet sich dadurch aus, dass darin - und nicht etwa im zugehörigen Defibrillator oder in einem zusätzlichen Gehäuse - ein Medikamentenspeicher vorgesehen ist. In Verbindung hiermit steht der Gedanke, dass der Anschluss der Elektrodenleitung als rein elektrischer Normanschluss ausgebildet, also kein Fluidanschluss zur Verbindung mit einem leitungs-externen Medikamentendepot vorhanden ist. Schließlich ist hinzuweisen auf den Gedanken, dass die Medikamentenabgabeeinrichtung(en) zur Ansteuerung durch einen über den elektrischen Anschluss in die Schockelektrodenleitung übertragenen elektrischen Impuls, und zwar insbesondere durch einen zugleich als Kardioversions- bzw. Schockimpuls dienenden Impuls, ausgebildet ist bzw. sind.

In einer Ausführung der Erfindung ist vorgesehen, dass der Medikamentenspeicher an oder nahe dem proximalen Ende der Schockelektrodenleitung angeordnet und über eine Fluidverbindung mit der oder jeder Medikamentenabgabeeinrichtung verbunden ist. Dies ermöglicht die Unterbringung einer relativ großen Medikamentenmenge, wie sie für eine oder mehrere medikamentös unterstützte Defibrillationen typischerweise erforderlich ist und die in Mikro-Hohlräumen von Medikamenten Dosierchips der weiter oben erwähnten Art nicht untergebracht werden kann. Als geeignete Bemessung der Depotgröße kann man eine solche ansehen, dass sie für 10 oder mehr und noch spezieller für 50 oder mehr Defibrillationen mit typischem Ablauf ausreicht.

Insbesondere ist hierbei der Medikamentenspeicher als zusammenhängender Hohlraum auf der Schockelektrodenleitung oder in einer Aufweitung derselben ausgebildet und mit einer selbst-versiegelnden Wandung zum Nachbefüllen im implantierten Zustand mittels einer Injektionsnadel versehen. Die hierdurch gegebene Möglichkeit eines - gegebenenfalls auch mehrfachen - Nachbefüllens des Medikamentendepots ermöglicht einen langjährigen Einsatz der Defibrillationsanordnung auch bei Patienten, bei denen relativ häufig Defibrillations-Behandlungen mit medikamentöser Unterstützung erforderlich werden.

In einer weiteren Ausführung der Erfindung ist vorgesehen, dass die Medikamentenabgabeeinrichtung eine elektrische Miniaturpumpe aufweist. In einer Ausgestaltung dieser Ausführungsform hat die Miniaturpumpe insbesondere einen piezoelektrischen Antrieb, und noch weiter bevorzugt ist die Nutzung der Energie eines Kardioversions- bzw. Defibrillationsimpulses als Antriebsenergie für jenen Antrieb.

In einer weiteren Ausführung der Erfindung ist eine Mehrzahl von Medikamentenabgabeeinrichtungen vorgesehen, die insbesondere längs des Leitungskörpers aufgereiht sind. Die Medikamentenabgabeeinrichtungen sind dabei insbesondere alle gemeinsam, oder jedenfalls in Gruppen gemeinsam, zur gleichzeitigen Ansteuerung mit dem elektrischen Anschluss der Leitung (und über diesen mit der Impulserzeugungseinheit des Kardioverters bzw. Defibrillators) verbunden und können hierdurch eine größere Medikamentendosis ausstoßen, als dies eine einzelne der miniaturisierten Einrichtungen könnte.

Eine differenzierte Steuerung einer zur elektrischen Impulsbeaufschlagung komplementären Medikamentenabgabe ermöglicht eine Ausführung, bei der die oder jede Medikamentenabgabeeinrichtung zur Freisetzung des Medikaments bei Anliegen einer Spannung oberhalb eines bestimmten Schwell-Pegels und/oder mit einer vorbestimmten Polarität ausgebildet ist. Je nach Vorgabe der Schockimpulspolarität bzw. -energie kann bei dieser Ausführung zusätzlich ein Medikament appliziert werden - oder eben nicht. Bei Verfügbarkeit von vorbestimmt programmierten Schockimpulsfolgen mit ansteigendem Spannungspegel und/oder sich ändernder Polarität kann auch ein Abschnitt der elektrischen Kardioversions-Therapie ohne, ein anderer Abschnitt hingegen mit Medikamentenabgabe erfolgen.

In einer weiteren Ausgestaltung dieses Gedankens sind in der Schockelektrodenleitung mindestens zwei separate Medikamentenspeicher und auf unterschiedliche elektrische Ansteuerung, insbesondere auf unterschiedliche Spannungspegel oder unterschiedliche Polarität eines Schockimpulses, ansprechende Medikamentenabgabeeinrichtungen in Verbindung mit den separaten Medikamentenspeichern vorgesehen. Dies ermöglicht es, in der Elektrodenleitung verschiedene kardioversions-unterstützende Medikamente zu speichern und auch in differenzierter Weise, in Abhängigkeit von den wesentlichen Parametern der ausgegebenen Kardioversions- bzw. Defibrillationsimpulse, an den Patienten abzugeben. In Zusammenhang mit einer geeigneten Sensorik des Defibrillators (die an sich bekannt ist und daher hier keiner weiteren Beschreibung bedarf) lassen sich differenzierte Therapieabläufe mit verschiedenen Medikamentengaben steuern.

Eine noch weitere Möglichkeit zur flexiblen Steuerung der Medikamentenabgabe bietet das Vorsehen von Mitteln zur Blockierung der Medikamentenabgabeeinrichtung(en) zur Inhibierung einer Medikamentenabgabe. Diese Mittel können eine Steuerleitung zur Verbindung mit dem Defibrillator und/oder einen Reed-Schalter zur Betätigung von außerhalb des Körpers aufweisen. Reed-Schalter als spezielle Magnetschalter implantierter Geräte zur Betätigung von außerhalb des Körpers sind an sich bekannt und kommerziell verfügbar, so dass auch diesbezüglich keine genauere Beschreibung erforderlich ist.

Eine erfindungsgemäße implantierbare Defibrillationsanordnung zeichnet sich, im Unterschied zu einigen weiter oben beschriebenen Anordnungen mit am Gerätekörper angebrachten Medikamentendosierchip, dadurch aus, dass Medikamentenabgabeeinrichtungen ausschließlich in der Schockelektrodenleitung vorgesehen sind. Daher kann am Applikationsort der elektrischen Impulse unmittelbar auch das die Elektrostimulation unterstützende Medikament appliziert werden und zugleich eine ineffiziente Medikamentenabgabe ungeeigneter Stelle des Körpers unterbleiben.

Im Hinblick auf die seit langem etablierten und weitgehend genormten ICD-Systeme ist es von Vorteil, dass die erfindungsgemäße Anordnung einen Standard-Defibrillator aufweist, der einen Ausgang zur Ausgabe von Schockimpulsen, aber keinen zusätzlichen Steuerausgang hat. Eine im Hinblick auf existierende Standards besonders sinnvolle Ausgestaltung sieht vor, dass der Anschluss zur Verbindung zwischen Schockelektrodenleitung und Defibrillator als Anschluss nach IS-1/DF-1 oder IS-4-Standard ausgeführt ist.

Vorteile und Zeckmäßigkeiten der Erfindung ergeben sich im Übrigen aus der nachfolgenden Beschreibung von Ausführungsbeispielen anhand der Figuren. Von diesen zeigen:
- Fig. 1: eine schematische Darstellung einer implantierten erfindungsgemäßen Defibrillationsanordnung
- Fig. 2: eine schematische Darstellung einer erfindungsgemäßen Schockelek- trodenleitung vor der Implantation, mit Einführbesteck und Führungs- draht,
- Fig.3: eine schematische Darstellung einer in der erfindungsgemäßen Schockelektrodenleitung einsetzbaren piezoelektrischen Medikamen- tenpumpe,
- Fig. 4: ein vereinfachtes Ablaufdiagramm des Betriebs einer erfindungsgemä- ßen Anordnung,
- Fig. 5: eine schematische Darstellung einer Schockelektrodenleitung gemäß einer weiteren Ausführungsform der Erfindung,
- Fig. 6: ein Flussdiagramm des Betriebs einer weiteren erfindungsgemäßen Defibrillationsanordnung, die eine Schockelektrodenleitung nach Fig. 5 umfasst, und
- Fig. 7a und 7b: schematische Ausschnittdarstellungen zweier Varianten einer weiteren Ausführungsform der erfindungsgemäßen Schockelektrodenleitung.

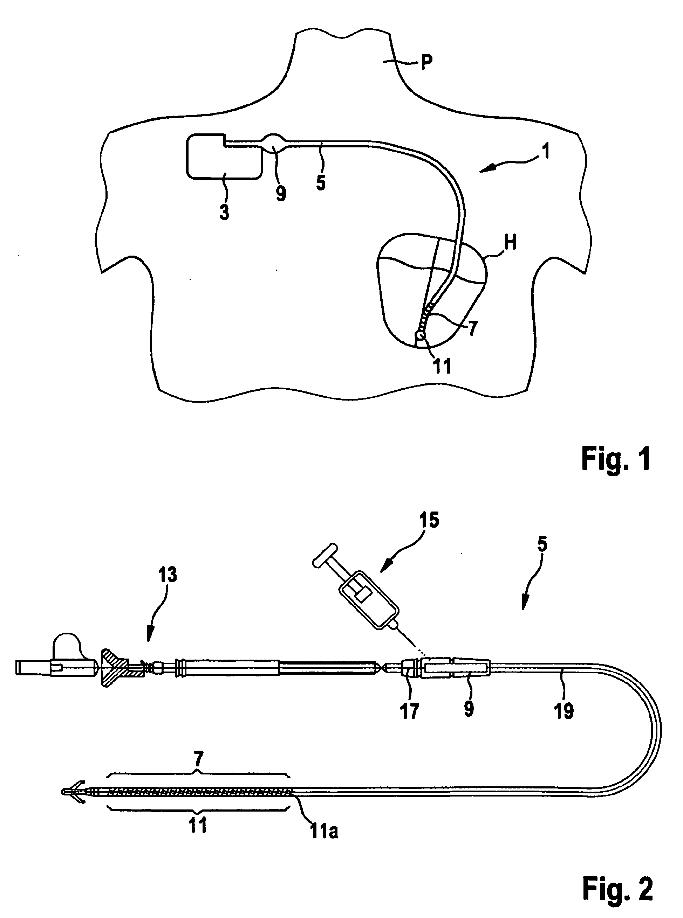

Fig. 1 zeigt eine implantierte Defibrillationsanordnung 1 mit einem implantierten Defibrillator (Energieversorgungs- und Steuereinheit) 3 und einer hieran angeschlossenen Schockelektrodenleitung 5, deren distales Ende in das Herz H eines Patienten P verlegt ist und dort, in Wandkontakt mit der Herzwand des Patienten, einen Schockelektrodenabschnitt 7 aufweist. Nahe dem proximalen Ende der Elektrodenleitung 5 ist in dieser ein Medikamentenspeicher 9 und am distalen Ende eine Medikamentenabgabeeinrichtung 11 angeordnet. Die Elektrodenleitung gewinnt hierdurch die kombinierten Eigenschaften einer elektrischen Defibrillationsleitung und eines Katheters zur defibrillations-unterstützenden Medikamentenabgabe.

Fig. 2 zeigt eine derartige Leitung 5 in der Startphase eines Implantationsvorganges, also in Verbindung mit einem Einführbesteck 13, sowie in synergistischer Darstellung mit einer Infusionsspritze 15 zum Wiederbefüllen des Medikamentenspeichers 9 etwas genauer. In Längsrichtung der Leitung 5 verläuft neben dem (nur während des Implantationsvorganges vorhandenen) Führungsdraht 17 eine Fluidverbindung (ein Medikamentenkanal) 19 zwischen dem Medikamentenspeicher 9 und der nahe dem distalen Ende der Leitung vorgesehenen Medikamentenabgabeeinrichtung 11, die hier mehrere Miniaturpumpen 11a umfasst. Wie die symbolische Darstellung der Infusionsspritze 15 zeigt, ist der Medikamentenspeicher 9 hier wiederbefüllbar ausgeführt, das heißt mit einer nach Einstichen selbst-verschließenden Wandung versehen und vorzugsweise mit einem geeigneten (nicht gezeigten) Röntgenmarker gekennzeichnet, um das Wiederbefüllen unter röntgenographischer Beobachtung leicht ausführen zu können.

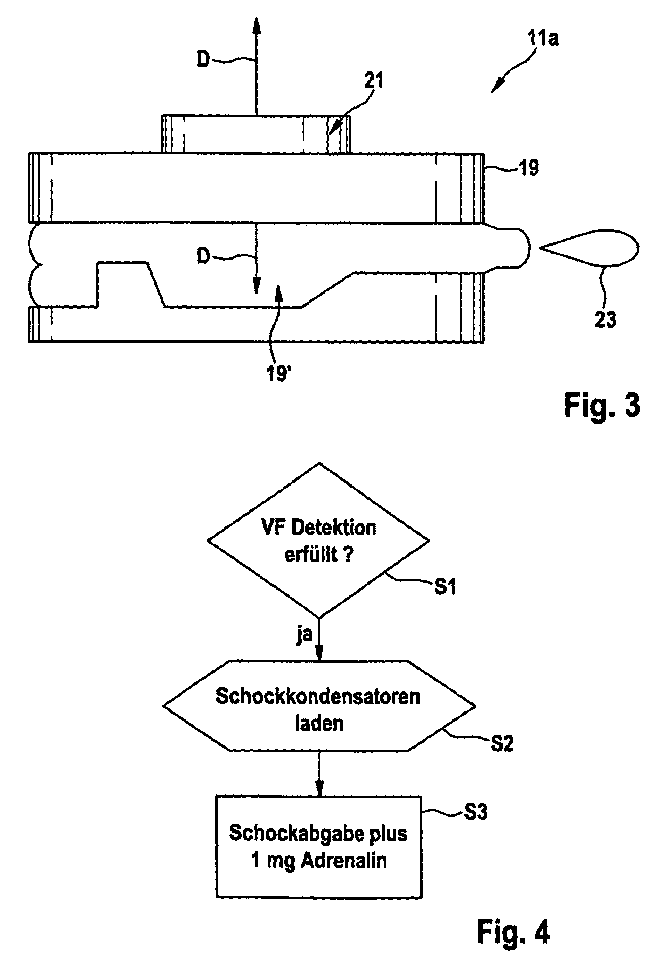

Fig. 3 zeigt als Beispiel einer Miniaturpumpe 11a schematisch eine auf piezoelektrischer Basis arbeitende Pumpe. An einem Pumpenkörper 19 sitzt hier ein Piezokristall 21, der durch (nicht dargestellte) stirnseitige Elektroden zu einer Verformung D angeregt werden kann, die in der Figur mit Pfeilen symbolisiert ist und in deren Folge die Wandung eines im Pumpenkörper 19 vorgesehenen Abschnitts 19' des Medikamentenkanals nach innen gedrückt wird. Im Ergebnis dieses Vorganges wird aus dem Kanalabschnitt 19' ein Medikamententröpfchen 23 ausgestoßen.

Es ist anzumerken, dass die Darstellung der Fig. 3 hochgradig schematisch ist und nicht den tatsächlichen Aufbau einer Piezo-Miniaturpumpe zeigen kann. Derartige Pumpen sind - etwa bei Tintenstrahldruckern - seit langem im technischen Einsatz und bedürfen daher hier keiner genaueren Beschreibung. Es ist auch darauf hinzuweisen, dass (ebenfalls beispielsweise von Tintenstrahldruckern) auch Arrays aus mehreren hintereinander gereihten Miniaturpumpen bekannt sind, die grundsätzlich ebenfalls zur Ausführung der vorliegenden Erfindung einsetzbar sind.

Für den Einsatz im Rahmen der Erfindung kann das Piezoelement 21 derart elektrisch verschaltet sein, dass ein Pol direkt mit der Schockelektrode 7, der andere aber mit einem Bezugspotential (beispielsweise auf der Ringelektrode der Elektrodenleitung) verbunden ist. Auf diese Weise wird die Miniaturpumpe 11a direkt mit der Schockenergie eines Kardioversions- bzw. Defibrillationsvorganges angetrieben, und es ist in vorteilhafter Weise kein separater Antrieb und keine spezielle Steuerung erforderlich.

Fig. 4 zeigt schematisch, wie ein kombinierter elektrischer Defibrillations- und Medikamentendosiervorgang mit einer Anordnung der in Fig. 1 gezeigten Art abläuft: Zunächst wird in einem Schritt S1 im Herzen erfasst, ob ein Zustand von Kammerflimmern (VF) vorliegt. Ist dies der Fall, werden in einem Schritt S2 Schockkondensatoren des Defibrillators 3 geladen, und nach Beendigung der Ladung wird in einem Schritt S3 ein Defibrillationsimpuls ausgegeben und durch diesen zugleich die Abgabe einer vorbestimmten Medikamentendosis über die Medikamentenausgabeeinrichtung(en) ausgeführt.

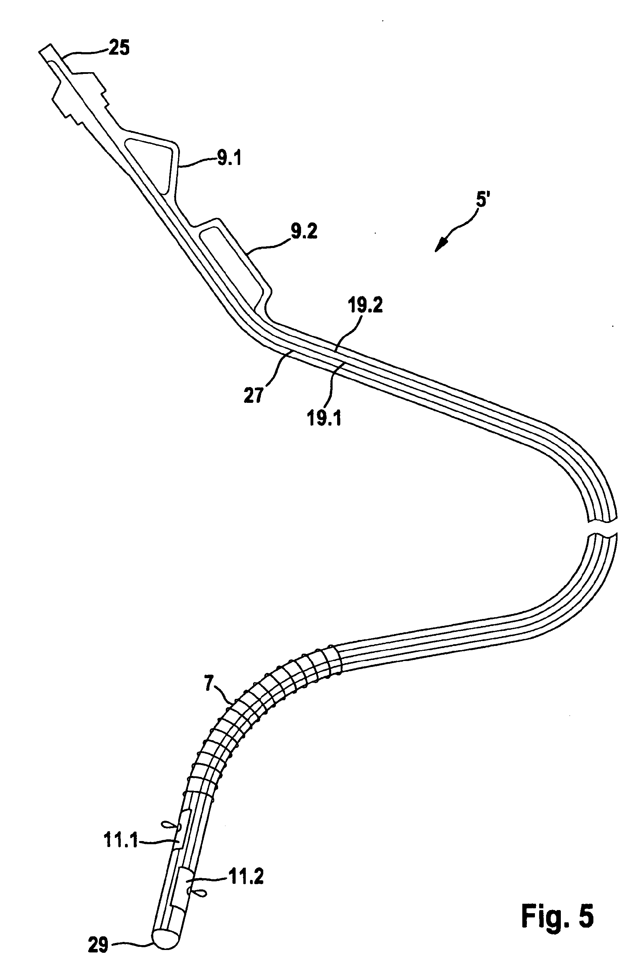

Eine differenziertere medikamentöse Unterstützung von Kardioversionsversuchen ist mit einer Schockelektrodenleitung 5' möglich, wie sie schematisch in Fig. 5 gezeigt ist. Nahe dem proximalen Ende und eines dort in üblicher Weise vorgesehenen elektrischen Anschlusssteckers 25 sind ein erster und zweiter Medikamentenspeicher 9.1, 9.2 vorgesehen, um zwei verschiedene kardioversions-unterstützende Medikamente bereitstellen zu können. In ihrer äußeren Form sind die beiden Medikamentenspeicher 9.1, 9.2 derart gefaltet, dass sie für ein Wiederbefüllen mittels einer Infusionsspritze tastbar und/oder auf dem Röntgenbild voneinander verschieden sind und nicht verwechselt werden können.

Längs des Leitungskörpers verläuft neben einer (hier vereinfacht als gestreckter Draht gezeigten, in der praktischen Ausführung aber typischerweise gewendelten) Elektrodenzuleitung 27 ein Paar von dünnen Fluidleitungen 19.1, 19.2, die den ersten und zweiten Medikamentenspeicher 9.1, 9.2, mit einer ersten und zweiten Medikamentenpumpe 11.1, 11.2 am distalen Ende der Leitung 5' verbinden. Die Elektrodenzuleitung 27 verbindet den Stecker 25 mit der Schockelektrode 7, und die Elektrode 7 ist ihrerseits (was in der Figur nur symbolisch dargestellt werden kann) mit Anschlüssen der beiden Pumpen 11.1. und 11.2 verbunden. Eine Tip-Elektrode 29 der Leitung 5' kann einerseits zum Abfühlen von Herzaktionspotentialen und somit zur Feststellung eines behandlungsbedürftigen Zustandes von Kammerflimmern dienen, ist aber außerdem mit Bezugspotentialanschlüssen der Pumpen 11.1 und 11.2 verbunden. (Die erforderliche Leitungsverbindung mit dem Stecker 25 ist in der Figur aus Gründen der besonderen Übersichtlichkeit fortgelassen).

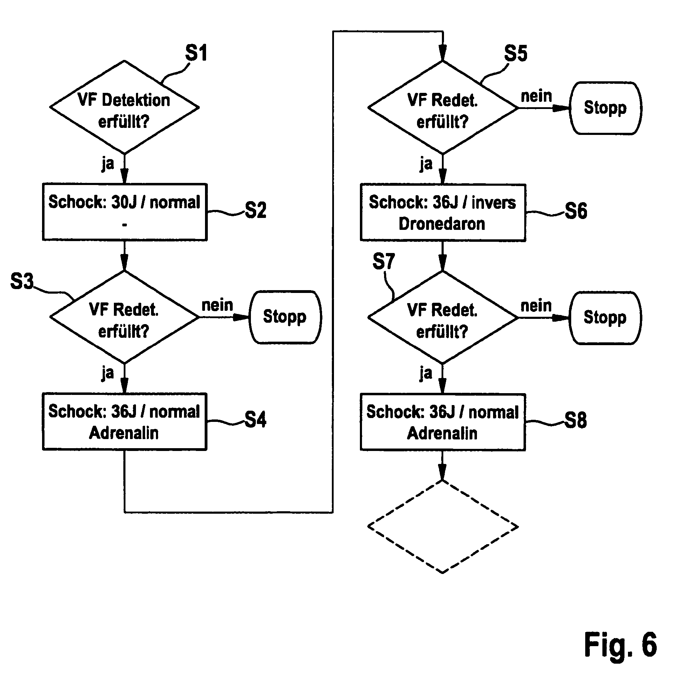

Die Funktion der in Fig. 5 gezeigten Elektrodenleitung wird in Verbindung mit dem in Fig. 6 dargestellten Flussdiagramm eines mehrschrittigen Defibrillations-Ablaufes beschrieben.

In dem in Fig. 6 dargestellten Ablaufplan wird eine mehrstufige medikamentöse Begleittherapie gemäß den derzeitigen Richtlinien zur Reanimation bei automatischer Defibrillation realisiert. Zunächst erfolgt eine VF-Detektion (S1) und Defibrillation mit einer Schockenergie kleiner der maximalen Schockenergie (S2) ohne Begleitmedikation. Sollte diese nicht erfolgreich sein, was in einem Schritt S3 ermittelt wird, erfolgt eine weitere Defibrillation mit maximaler Schockenergie, normaler Schockpolarität und gleichzeitiger intrakardialer Applikation von Adrenalin (S4). Ist auch diese Therapie nicht erfolgreich, was in einem Schritt S5 ermittelt wird, erfolgt die nächste Schockabgabe mit maximaler Schockenergie (S6), diesmal aber mit inverser Schockpolarität und intrakardialer Gabe von Dronedaron (alternativ: Amiodaron). In einem Erfassungsschritt S7 wird auch hierbei das Ergebnis überprüft, und bei Misserfolg werden bis zu 3 weitere Maximalenergieschocks normaler Polarität mit Gabe von Adrenalin abgegeben (S8).

Die Steuerung der Medikamentenpumpe erfolgt in diesem Ausführungsbeispiel über die Schockenergie und Schockpolarität. Die Medikamentenpumpe ist hier um eine polaritäts- und spannungsabhängige Triggereinheit ergänzt, so dass bei maximaler Schockenergie und normaler Polarität Adrenalin und bei maximaler Schockenergie und inverser Polarität Dronedaron appliziert wird. Ist die Schockenergie kleiner als Maximalenergie, erfolgt keine Medikamentenabgabe.

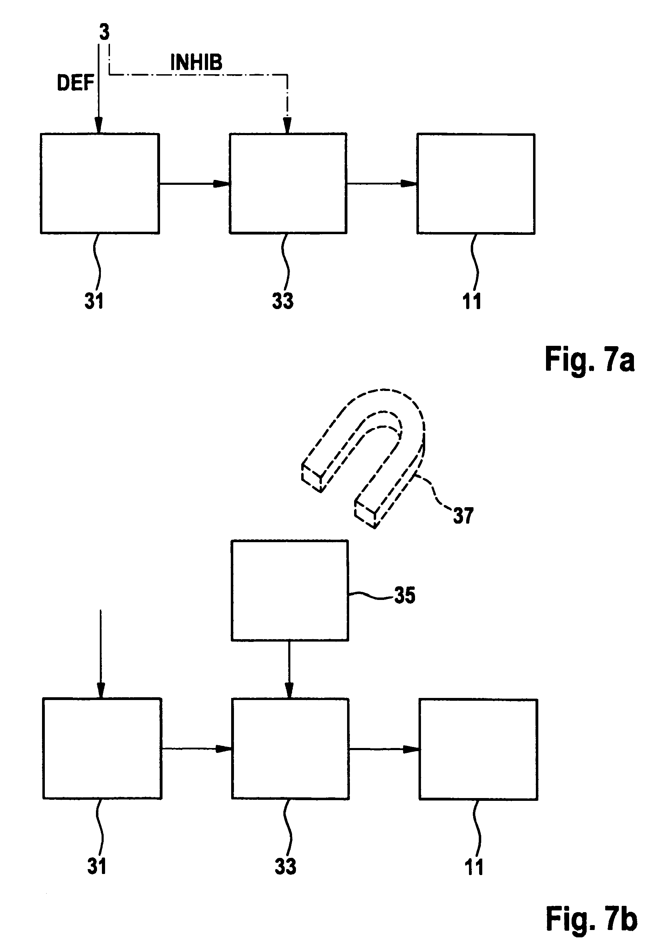

Fig. 7a und 7b zeigen schematisch, in Art eines Funktions-Blockschaltbildes, zwei Möglichkeiten für die wahlweise Inhibierung einer Medikamentenabgabe bei einem Kardioversions- bzw. Defibrillationsvorgang. In beiden dargestellten Anordnungen ist zwischen eine Medikamtenabgabeeinrichtung 11 und eine zugeordnete Antriebsquelle 31, die ihrerseits ein Steuersignal DEF von dem zur Anordnung gehörenden Kardioverter/Defibrillator erhält, eine Inhibierungsstufe 33 geschaltet. Bei der Anordnung nach Fig. 7a erhält diese von Defibrillator 3 im Inhibierungs-Fall ein Steuersignal INHIB, und dieses bewirkt eine Aktivierung der Inhibierungsstufe und damit eine Unterbrechung der Verbindung zwischen Antrieb und Medikamentenabgabeeinrichtung. Bei der Ausführung nach Fig. 7b wird dies durch einen Reed-Kontakt 35 bewirkt, der von außerhalb des Körpers durch einen Magneten 37 betätigt werden kann.

Die Ausführung der Erfindung ist nicht auf die oben beschriebenen Beispiele und hervorgehobenen Aspekte beschränkt, sondern ebenso in einer Vielzahl von Abwandlungen möglich, die im Rahmen fachgemäßen Handelns liegen.

## Patentansprüche

1. Implantierbare Schockelektrodenleitung (5) mit einem proximalen Anschluss zur Verbindung mit einem implantierbaren Defibrillator (3), einem langgestreckten flexiblen Leitungskörper und einer an oder nahe einem distalen Ende des Leitungskörpers angeordneten Schockelektrode (7) sowie einer Medikamentenabgabeeinrichtung (11) wobei in der Schockelektrodenleitung ein mit der Medikamentenabgabeeinrichtung verbundener Medikamentenspeicher (9) vorgesehen ist **dadurch gekennzeichnet daß** der Anschluss als ein rein elektrischer Normanschluss ausgebildet ist und wobei die Medikamentenabgabeeinrichtung (11) zur Ansteuerung durch einen über den elektrischen Anschluss in die Schockelektrodenleitung übertragenen elektrischen Impuls ausgebildet ist.

2. Schockelektrodenleitung nach Anspruch 1, wobei der Medikamentenspeicher (9) an oder nahe dem proximalen Ende der Schockelektrodenleitung (5) angeordnet und über eine Fluidverbindung mit der oder jeder Medikamentenabgabeeinrichtung (11) verbunden ist.

3. Schockelektrodenleitung (5) nach Anspruch 1 oder 2, wobei der Medikamentenspeicher (9) als zusammenhängender Hohlraum auf der Schockelektrodenleitung oder in einer Aufweitung derselben ausgebildet und mit einer selbst-versiegelnden Wandung zum Nachbefüllen im implantierten Zustand mittels einer Injektionsnadel versehen ist.

4. Schockelektrodenleitung (5) nach einem der vorangehenden Ansprüche, wobei die Medikamentenabgabeeinrichtung (11) eine elektrische Miniaturpumpe aufweist.

5. Schockelektrodenleitung (5) nach Anspruch 4, wobei die Miniaturpumpe einen piezoelektrischen Antrieb aufweist, dessen Antriebsenergie durch einen vom Defibrillator abgebenden Schockimpuls gebildet wird.

6. Schockelektrodenleitung (5) nach einem der vorangehenden Ansprüche, wobei eine Mehrzahl von Medikamentenabgabeeinrichtungen (11) vorgesehen ist, die insbesondere längs des Leitungskörpers aufgereiht sind.

7. Schockelektrodenleitung (5) nach Anspruch 6, wobei die oder mindestens eine Medikamentenabgabeeinrichtungen (11) jeweils gemeinsam zur gleichzeitigen Ansteuerung mit dem elektrischen Anschluss verbunden sind.

8. Schockelektrodenleitung (5) nach einem der vorangehenden Ansprüche, wobei die oder jede Medikamentenabgabeeinrichtung (11) zur Freisetzung des Medikaments bei Anliegen einer Spannung oberhalb eines bestimmten Schwell-Pegels und/oder mit einer vorbestimmten Polarität ausgebildet ist.

9. Schockelektrodenleitung (5) nach einem der vorangehenden Ansprüche, wobei mindestens zwei separate Medikamentenspeicher und auf unterschiedliche elektrische Ansteuerung, insbesondere auf unterschiedliche Spannungspegel oder unterschiedliche Polarität eines Schockimpulses, ansprechende Medikamentenabgabeeinrichtungen in Verbindung mit den separaten Medikamentenspeichem vorgesehen sind.

10. Schockelektrodenleitung (5) nach einem der vorangehenden Ansprüche, wobei der oder jeder Medikamentenspeicher (9) zur Aufnahme für eine Vielzahl von, insbesondere 10 oder mehr oder noch spezieller 50 oder mehr, Defibrillationen ausreichende Medikamentenmenge bemessen ist.

11. Schockelektrodenleitung (5) nach einem der vorangehenden Ansprüche, wobei Mittel zur Blockierung der oder jeder Medikamentenabgabeeinrichtung (11) zur selektiven Inhibierung einer Medikamentenabgabe vorgesehen sind.

12. Schockelektrodenleitung (5) nach Anspruch 11, wobei die Mittel zur Blockierung eine Steuerleitung zur Verbindung mit dem Defibrillator (3) und/oder einen Reed-Schalter zur Betätigung von außerhalb des Körpers aufweisen.

13. Implantierbare Defibrillationsanordnung (1) mit einem implantierbaren Defibrillator (3) und einer Schocketektrodenieitung (5) nach einem der vorangehenden Ansprüche, wobei eine Medikamentenabgabeeinrichtung (11) ausschließlich in der Schockelektrodenleitung (5) vorgesehen ist.

14. Defibrillationsanordnung (1) nach Anspruch 13, welche einen Standard-Defibrillator aufweist (3), der einen Ausgang zur Ausgabe von Schockimpulsen, aber keinen zusätzlichen Steuerausgang hat.

15. Defibrillationsanordnung (1) nach Anspruch 13 oder 14, wobei der Anschluss zur Verbindung zwischen Schockelektrodenleitung (5) und Defibrillator (3) als Anschluss nach IS-1/DF-1 oder IS-4-Standard ausgeführt ist.

## Claims

1. An implantable shock electrode line (5), comprising a proximal terminal for the connection to an implantable defibrillator (3), an elongated flexible line body, and a shock electrode (7) disposed on or close to a distal end of the line body, and further comprising a drug delivery device (11), a drug reservoir (9) connected to the drug delivery device being provided in the shock electrode line, **characterized in that** the terminal is designed as a purely electrical regular terminal, and the drug delivery device (11) is designed to be actuated by an electric pulse transmitted by way of the electric terminal into the shock electrode line.

2. The shock electrode line according to claim 1, wherein the drug reservoir (9) is disposed on or close to the proximal end of the shock electrode line (5) and connected to the, or each, drug delivery device (11) by way of a fluid connection.

3. The shock electrode line (5) according to claim 1 or 2, wherein the drug reservoir (9) is designed as a continuous hollow space on the shock electrode line or in an expansion thereof and provided with a self-sealing wall for replenishment in the implanted state by means of an injection needle.

4. The shock electrode line (5) according to any one of the preceding claims, wherein the drug delivery device (11) comprises an electric miniature pump.

5. The shock electrode line (5) according to claim 4, wherein the miniature pump comprises a piezo-electric drive, the drive energy of which is formed by a shock pulse delivered by the defibrillator.

6. The shock electrode line (5) according to any one of the preceding claims, wherein a plurality of drug delivery devices (11) are provided, which are stringed in particular along the line body.

7. The shock electrode line (5) according to claim 6, wherein the, or at least one of the, drug delivery devices (11) in each case are jointly connected to the electrical terminal for simultaneous actuation.

8. The shock electrode line (5) according to any one of the preceding claims, wherein the, or each, drug delivery device (11) is designed to release the drug when a voltage is present that exceeds a defined threshold level and/or has a predetermined polarity.

9. The shock electrode line (5) according to any one of the preceding claims, wherein at least two separate drug reservoirs and drug delivery devices, which respond to different electrical actuation, in particular to different voltage levels or different polarity of a shock pulse, are provided in conjunction with the separate drug reservoirs.

10. The shock electrode line (5) according to any one of the preceding claims, wherein the, or each, drug reservoir (9) is dimensioned to receive a drug quantity that is sufficient for a plurality of defibrillations, in particular 10 or more, or even more particularly 50 or more.

11. The shock electrode line (5) according to any one of the preceding claims, wherein means for blocking the, or each, drug delivery device (11) are provided for selectively inhibiting a drug delivery.

12. The shock electrode line (5) according to claim 11, wherein the means for blocking comprise a control line for the connection to the defibrillator (3) and/or a reed switch for actuation outside of the body.

13. An implantable defibrillation arrangement (1), comprising an implantable defibrillator (3) and a shock electrode line (5) according to any one of the preceding claims, wherein a drug delivery device (11) is provided exclusively in the shock electrode line (5).

14. The defibrillation arrangement (1) according to claim 13, which comprises a standard defibrillator (3) that has an output for putting out shock pulses, but no additional control output.

15. The defibrillation arrangement (1) according to claim 13 or 14, wherein the terminal for the connection between the shock electrode line (5) and the defibrillator (3) is designed as a terminal according to the IS-1/DF-1 or IS-4 standard.

## Revendications

1. Câble d'électrode de choc implantable (5) comprenant une connexion proximale pour une liaison avec un défibrillateur implantable (3), un corps de câble flexible allongé et une électrode de choc (7) disposée à ou près d'une extrémité distale du corps de câble (7) ainsi qu'un dispositif d'administration de médicament (11), un dispositif de stockage de médicament (9) relié au dispositif d'administration de médicament étant prévu dans le câble d'électrode de choc, **caractérisé par le fait que** la connexion est sous la forme d'une connexion électrique normée nette et le dispositif d'administration de médicament (11) est conçu pour être commandé par une impulsion électrique chargée par la connexion électrique dans le câble d'électrode de choc.

2. Câble d'électrode de choc selon la revendication 1, dans lequel le dispositif de stockage de médicament (9) est disposé à ou près de l'extrémité proximale du câble d'électrode de choc (5) et est lié par une liaison fluidique avec le dispositif d'administration de médicament (11) ou avec chacun des dispositifs d'administration de médicament (11).

3. Câble d'électrode de choc (5) selon la revendication 1 ou 2, dans lequel le dispositif de stockage de médicament (9) prend la forme d'un espace creux cohérent sur le câble d'électrode de choc ou dans un élargissement de celui-ci et comprend une paroi auto-scellante pour un re-remplissage dans l'état implanté au moyen d'une aiguille d'injection.

4. Câble d'électrode de choc (5) selon l'une des revendications précédentes, dans lequel le dispositif d'administration de médicament (11) comprend une pompe électrique miniature.

5. Câble d'électrode de choc (5) selon la revendication 4, dans lequel la pompe miniature comprend une commande piézoélectrique, dont l'énergie de commande est formée par une impulsion de choc émise par le défibrillateur.

6. Câble d'électrode de choc (5) selon l'une des revendications précédentes, dans lequel une pluralité de dispositifs de distribution de médicament (11) est prévue, qui sont en particulier alignés le long du corps de câble.

7. Câble d'électrode de choc (5) selon la revendication 6, dans lequel les ou au moins l'un des dispositifs d'administration de médicament (11) sont tous reliés ensemble à une commande simultanée par la connexion électrique.

8. Câble d'électrode de choc (5) selon l'une des revendications précédentes, dans lequel les ou chaque dispositif d'administration de médicament (11) est conçu pour la libération du médicament par l'application d'une tension au-dessus d'un niveau seuil déterminé et/ou avec une polarité prédéterminée.

9. Câble d'électrode de choc (5) selon l'une des revendications précédentes, dans lequel on prévoit au moins deux dispositifs de stockage de médicament séparés et des dispositifs d'administration de médicament correspondants en liaison avec les dispositifs de stockage de médicament séparés, les dispositifs d'administration de médicament étant sous une commande électrique différente, en particulier sous un niveau de tension différent ou sous une polarité différente d'une impulsion de choc.

10. Câble d'électrode de choc (5) selon l'une des revendications précédentes, dans lequel le ou chaque dispositif de stockage de médicament (9) est mesuré pour recevoir une quantité de médicament suffisante pour une pluralité de défibrillations, en particulier 10 défibrillations ou plus ou encore de façon plus spéciale 50 défibrillations ou plus.

11. Câble d'électrode de choc (5) selon l'une des revendications précédentes, dans lequel sont prévus des moyens de blocage du ou de chaque dispositif d'administration de médicament (11) pour l'inhibition sélective d'une administration de médicament.

12. Câble d'électrode de choc (5) selon la revendication 11, dans lequel les moyens de blocage comprennent un câble de commande pour une liaison avec le défibrillateur (3) et/ou un commutateur Reed pour l'actionnement de l'extérieur du corps.

13. Ensemble de défibrillation implantable (1) comprenant un défibrillateur implantable (3) et un câble d'électrode de choc (5) selon l'une des revendications précédentes, dans lequel est prévu un dispositif d'administration de médicament (11) exclusivement dans le câble d'électrode de choc (5).

14. Système de défibrillation (1) selon la revendication 13, qui comprend un défibrillateur standard (3) qui a une sortie pour l'émission d'impulsions de choc mais pas de sortie de commande additionnelle.

15. Système de défibrillation (1) selon la revendication 13 ou 14, dans lequel la connexion de liaison entre le câble d'électrode de choc (5) et le défibrillateur (3) prend la forme d'une connexion selon le standard IS-1/DF-1 ou IS-4.
